# EUROPEAN PATENT APPLICATION

(11) **EP 4 656 152 A1**
(43) Date of publication of application: **03.12.2025**
(21) Application number: 25178687.7
(22) Date of filing: 26.05.2025
(51) Int. Cl.: A61B 34/10, A61B 34/00, G06T 19/20, A61B 34/20, A61B 90/00

(54) **SYSTEM AND METHOD OF DEFINING AND DISPLAYING ALERT ZONES OF A PATIENT ANATOMY**

(30) Priority: 30.05.2024 US 202463653451 P
(71) Applicant: Stryker European Operations Limited, Carrigtwohill, Co. Cork T45 HX08 (IE)
(72) Inventor: Hertel, Julius Maximilian, 79114 Freiburg (DE)
(74) Representative: Schott, Jakob Valentin

(57) **Abstract**

A surgical navigation system is provided. The surgical navigation system comprises a display device and a memory device containing a volumetric image, which includes a volumetric representation of the patient anatomy. The surgical navigation system also comprises a controller in communication with the display and the memory device, the controller configured to define a portion of volumetric representation as an alert boundary, define a buffer surface surrounding the alert boundary and offset from the alert boundary by a predefined buffer distance, define a buffer zone as a portion of the volumetric image enclosed by the buffer surface, define a portion of the buffer zone as a visualized portion of the buffer zone, and control the display device to display the visualized portion of the buffer zone overlaid on the volumetric representation of the patient anatomy.

## Description

### TECHNICAL FIELD

The present disclosure relates generally to systems and methods related to the display of three-dimensional images of a patient.

### BACKGROUND

During medical procedures where the anatomy of a patient is treated, it is important for a surgical system to warn an operator of critical anatomical structures of the anatomy to be avoided. For instance, during a medical procedure involving the treatment of bone, certain portions of the bone may be adjacent to critical anatomical structures, such as cortical walls, nerves, blood vessels, and should be avoided during the medical procedure. Currently, there exists a need in the art to allow an operator to select which portions of the anatomy should be avoided during a medical procedure. Additionally, there exists a need in the art to clearly and accurately present the portions of the anatomy to be avoided to an operator performing the medical procedure.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical navigation system is provided. The surgical navigation system comprises: a display device; a memory device containing a volumetric image, the volumetric image including a volumetric representation of a patient anatomy, the volumetric representation of the patient anatomy having an outer surface; and a controller in communication with the display and the memory device, the controller configured to: define at least a portion of the outer surface as an alert boundary, define a buffer surface surrounding the alert boundary and offset from the alert boundary by a predefined buffer distance, define a buffer zone as a portion of the volumetric image enclosed by the buffer surface, define at least a portion of the buffer zone as a visualized portion of the buffer zone, wherein the visualized portion of the buffer zone intersects the volumetric representation of the patient anatomy, and control the display device to display at least a portion of the visualized portion of the buffer zone overlaid on at least a portion of the volumetric representation of the patient anatomy.

According to a second aspect, a method of operating a surgical navigation system including a display device is provided. The method comprises steps of: generating a volumetric image including a volumetric representation of a patient anatomy, the volumetric representation of the patient anatomy having an outer surface; defining at least a portion of the outer surface as an alert boundary, defining a buffer surface surrounding the alert boundary and offset from the alert boundary by a predefined buffer distance, defining a buffer zone as a portion of the volumetric representation of the patient anatomy enclosed by the buffer surface, defining at least a portion of the buffer zone as a visualized portion of the buffer zone, wherein the visualized portion of the buffer zone intersects the volumetric representation of the patient anatomy, and displaying, with the display device, at least a portion of the visualized portion of the buffer zone overlaid on at least a portion of the volumetric representation of the patient anatomy.

According to a third aspect, a surgical navigation system is provided. The surgical navigation system comprising: a display device; a memory device containing a volumetric image, the volumetric image including a plurality of voxels and a volumetric representation of a patient anatomy, the volumetric representation of the patient anatomy having an outer surface; and a controller in communication with the display and the memory device, the controller configured to: generate an anatomy distance field corresponding to the volumetric image, wherein the anatomy distance field includes a plurality of anatomy distance values, each anatomy distance value being assigned to a voxel of the volumetric image; generate an alert zone distance field corresponding to the volumetric image, wherein the alert zone distance field includes a plurality of alert zone distance values, each alert zone distance value being assigned to a voxel of the volumetric image; assign a signed anatomy distance value to a voxel of the volumetric image within the outer surface; assign an unsigned alert zone distance value to a voxel of the volumetric image based on a distance of the voxel from the alert boundary; and define the alert zone based on determining voxels of the volumetric image assigned a signed anatomy distance value and an unsigned alert zone distance value less than a threshold value.

According to a fourth aspect, a method of operating a surgical navigation system is provided. The method comprising steps of: generating an anatomy distance field corresponding to the volumetric image, wherein the anatomy distance field includes a plurality of anatomy distance values, each anatomy distance value being assigned to a voxel of the volumetric image; generating an alert zone distance field corresponding to the volumetric image, wherein the alert zone distance field includes a plurality of alert zone distance values, each alert zone distance value being assigned to a voxel of the volumetric image; assigning a signed anatomy distance value to a voxel of the volumetric image within the outer surface; assigning an unsigned alert zone distance value to a voxel of the volumetric image based on a distance of the voxel from the alert boundary; and defining the alert zone based on determining voxels of the volumetric image assigned a signed anatomy distance value and an unsigned alert zone distance value less than a threshold value.

Any of the aspects can be combined in part or in whole. Any of the aspects can be combined in part or in whole with any of the following implementations:

In some implementations, the volumetric representation of the patient anatomy may be generated by performing segmentation on a CT image of the patient anatomy.

In some implementations, the surgical navigation system further comprises a user input device configured to receive an identification of the alert boundary. In some implementations, the user input device may be configured to receive a selected portion of the outer surface as the identification of the alert boundary, and the selected portion of the outer surface may be defined as the alert boundary.

In some implementations, a portion of the outer surface may be identified as corresponding to an anatomical landmark of the patient anatomy. In some implementations, the portion of the outer surface corresponding to the anatomical landmark of the patient anatomy may be defined as the alert boundary.

In some implementations, the volumetric image includes a plurality of voxels.

In some implementations, an anatomy distance field corresponding to the volumetric image may be generated, wherein the anatomy distance field includes a plurality of anatomy distance values, each anatomy distance value being assigned to a voxel of the volumetric image. In some implementations, the voxels of the volumetric image within the outer surface are identified. In some implementations, a signed anatomy distance value may be assigned to a voxel of the volumetric image within the outer surface.

In some implementations, an alert zone distance field corresponding to the volumetric image may be generated, wherein the alert zone distance field includes a plurality of alert zone distance values, each alert zone distance value being assigned to a voxel of the volumetric image. In some implementations, an unsigned alert zone distance value may be assigned to a voxel of the volumetric image based on a distance of the voxel from the alert boundary. In some implementations, an interpolation value may be calculated for each voxel based on the anatomy distance value and alert zone distance value assigned to the voxel.

In some implementations, the visualized portion of the buffer zone may be defined based on determining voxels of the volumetric image assigned a signed anatomy distance value and an unsigned alert zone distance value less than a threshold value. In some implementations, the threshold value may be based on the predefined buffer distance. In some implementations, the threshold value may be based on a size of the alert zone.

In some implementations, the method may be carried out by instructions stored on the computer readable storage medium.

### BRIEF DESCRIPTION OF THE DRAWINGS

Advantages of the present invention will be readily appreciated as the same becomes better understood by reference to the following detailed description when considered in connection with the accompanying drawings.
FIG. 1 is a perspective view of an exemplary layout of an operating room including a display device, at least one surgical instrument assembly, and a surgical navigation system for performing a medical procedure on a patient.
FIG. 2 is a perspective view of a patient anatomy, where a portion of the patient anatomy is defined as an alert zone.
FIG. 3 is a perspective view of a volumetric image including a volumetric representation of the patient anatomy of FIG. 2.
FIG. 4 is a flowchart illustrating an exemplary method of defining and displaying an alert zone.
FIG. 5A is a schematic view illustrating steps of the method of FIG. 4 of defining the alert zone of the method of FIG. 4.
FIG. 5B is a schematic view illustrating steps of the method of FIG. 4 of displaying the alert zone.
FIG. 6 is a schematic view of an exemplary anatomy distance field.
FIG. 7 is a schematic view of an exemplary alert zone distance field.
FIG. 8 is a schematic view of the anatomy distance field and the alert zone distance field of FIGS. 6 and 7.

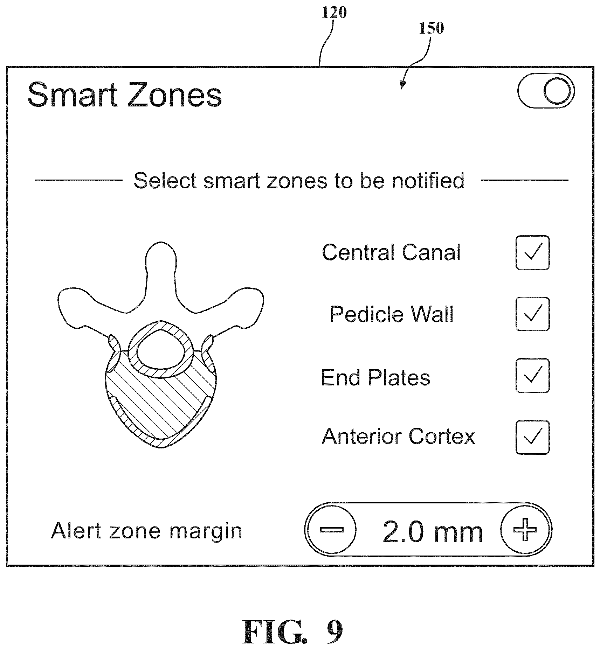

FIG. 9 is an exemplary GUI of the display device of FIG. 1, where a user may select a portion of the anatomy as an alert zone via the GUI.
FIG. 10A is a schematic view of a buffer zone overlaid onto a volumetric representation of a patient anatomy, wherein an alert zone is defined based on the buffer zone.
FIG. 10B is a schematic view of an alert zone overlaid onto the volumetric representation of the patient anatomy of FIG. 10A, wherein the alert zone is defined based on the buffer zone of FIG. 10A, and wherein the alert zone includes a pixelated outline.
FIG. 10C is a schematic view of an alert zone overlaid onto the volumetric representation of the patient anatomy of FIG. 10A, wherein the alert zone is defined based on the buffer zone of FIG. 10A, and wherein the alert zone includes a continuous outline.
FIGS. 11A-11D are exemplary displays of alert zones and the volumetric representation of the patient anatomy.

### DETAILED DESCRIPTION

### I. Example System Overview

Referring to the Figures, wherein like numerals indicate like or corresponding parts throughout the several views, a surgical system 100 and methods for using the same are shown throughout.

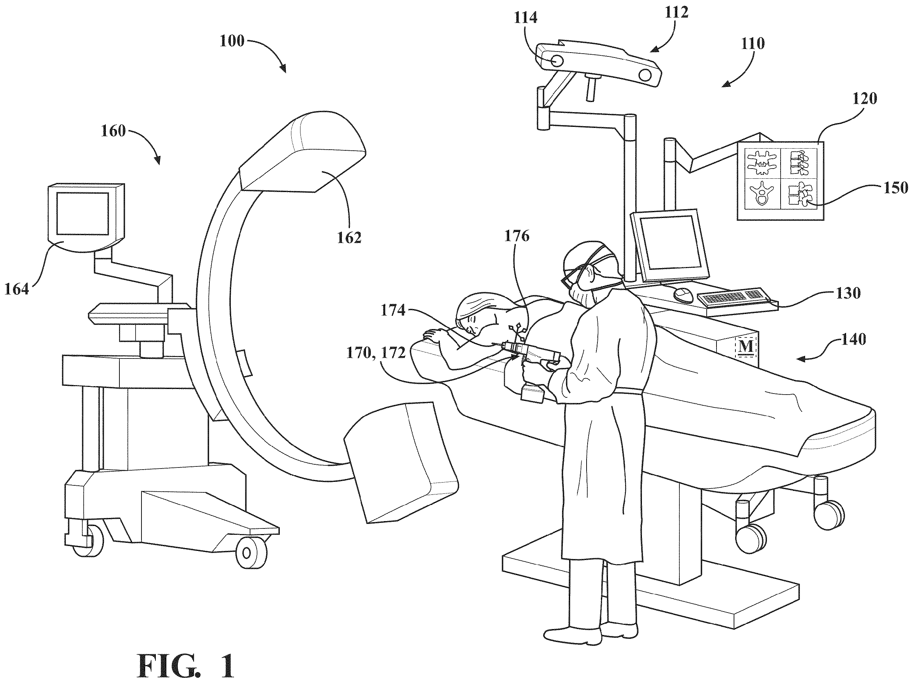

Referring to FIG. 1, an exemplary configuration of an operating room or surgical suite for performing a medical procedure on a patient using the surgical system 100 is shown. The medical procedure may be any procedure for treating any suitable anatomy of a patient, such as bone or soft tissue. For example, in FIG. 1, the patient is undergoing a medical procedure where the spine and vertebra of the patient are being treated. The surgical procedure may involve tissue removal or treatment. The surgical system 100 described herein may be utilized for treating any anatomical structure(s), for example, such as joints, including knee joints, hip joints, shoulder joints, ankles joints, or any other bone structure(s) not described herein. The surgical system 100 can be used to perform any type of procedure, including any spinal procedure, partial knee arthroplasty, total knee arthroplasty, total hip arthroplasty, anatomical shoulder arthroplasty, reverse shoulder arthroplasty, fracture repair surgery, osteotomies, and the like. Similarly, the techniques and methods described herein can be used with any type of robotic system and for any procedure.

As shown in FIG. 1, the surgical system 100 may include a surgical navigation system 110. The surgical navigation system 110 may include a navigation controller 140, a display unit 120, and a tracking unit 112.

The navigation controller 140 may be any suitable controller for implementing the various routines, functions, or methods disclosed herein. The navigation controller 140 may include a central processing unit (CPU) and/or other processors. Additionally, the navigation controller 140 may include and/or be in communication with a memory M. The navigation controller 140 may be a personal computer, laptop computer, tablet computer or any other suitable computing device. The navigation controller 140 may include surgical navigation software including one or more modules and/or operating instructions related to the operation of the surgical navigation system 110 and to implement the various routines, functions, or methods disclosed herein. For example, the navigation controller 140 may be configured to receive input from user input devices 130, generate/update various graphical user interfaces (GUI) for display by the display unit 120, and/or perform segmentation on patient images. The navigation controller 140 may also generate a surgical plan for a medical procedure by defining a surgical pathway and/or planned poses of a medical device. The navigation controller 140 may also generate the surgical plan by defining virtual boundaries, virtual constraints, planned trajectories for constraining the medical device.

The display unit 120 may be configured to display various GUIs 150 and patient images (e.g., pre-operative patient images or intraoperative patient images). The pre-operative images may be uploaded to the surgical navigation system 110 prior to the surgical procedure. A user such as a medical professional may interact with the various GUIs 150 via user input devices 130 or via touch input. The display unit 120 of the surgical navigation system 110 may be configured to display various prompts or data entry boxes. For example, the display unit 120 may be configured to display a text box or prompt that allows the user to manually enter or select the type of surgical procedure to be performed.

The display unit 120 may be further configured to display a surgical plan for a medical procedure overlaid on the patient images. The surgical plan may include the surgical pathway for executing the medical procedure, planned trajectory, orientation, and/or position for the medical instrument and/or implant during the medical procedure. The surgical plan may also include a pose of an implant or medical device to be inserted during the medical procedure overlaid onto the patient data or image. It is contemplated that the surgical navigation system 110 may be configured to display and/or project a holographic image of surgical pathway for executing the medical procedure or planned trajectory or orientation for the medical instrument during the medical procedure. This may include projecting the surgical pathway onto the patient or other surface in the operating room. It may also include a projection of the surgical pathway onto the head unit worn by the user, such as a lens, shield, or glasses of the head unit. An exemplary configuration of the surgical navigation system 110 including a display unit worn by the user to display the target trajectory and/or target location is disclosed in International Publication No. WO 2018/203304 A1, entitled "Surgical Navigation System", the entirety of which is hereby incorporated by reference.

The navigation controller 140 may receive input from the user via the user input devices 130 and/or the GUI 150.

In some instances, the user may enter patient data via the user input devices 130 and/or the GUI 150. The patient data, in addition to the patient images, may include additional information related to the type of medical procedure being performed, the patient's anatomical features, the patient's specific medical condition, and/or operating settings for the surgical navigation settings. For instance, the user may input various anatomical dimensions related to the patient anatomy. The user may also identify and/or select anatomical features from the patient data. This may include selecting the surgical site, such as selecting the anatomical structure and/or specific area on the anatomical structure where the medical procedure is to be performed. In an example instance, in performing a spinal fusion procedure, the user may enter information via the user input devices 130 and/or the GUI 150 related to specific vertebra or vertebra on which the medical procedure is being performed.

In some instances, the user may enter information related to the surgical plan via the user input devices 130 and/or the GUI 150. For example, the user may input, via the user input devices 130 and/or the GUI 150, the size and shape of a medical device or implant to be inserted during the medical procedure. As another example, the input to the user input devices 130 or to the GUI 150 may be provided to select the surgical instrument to be used, to select the device and/or implant to be inserted, to select a planned pose where the device or implant is to be placed within the patient, and to allow the user to select the parameters of the implant to be inserted, such as the length and/or diameter of the screw to be inserted. As yet another example, the user may provide input to the user input devices 130 or to the GUI 150 to select and/or input a target location, target trajectory, target depth or similar feature of the surgical plan to help guide the user in performing the medical procedure.

The surgical system 100 may also include an imaging system 160 in communication with the surgical navigation system 110. The imaging system 160, such as CT or MRI imaging device, may perform intraoperative imaging. If the imaging system 160 is a CT imaging device, the imaging system 160 may generate CT image data. The imaging system 160 may include a scanner 162 and a display unit 164. The scanner 162 may be utilized to take an image of the patient and display it on the display unit 164. For example, the scanner 162 may include a C-arm configured to be rotated about the patient to produce a plurality of images of the patient. The imaging system 160 may also include a processor including software, as is known by those skilled in the art, which is capable of taking the plurality of images captured by the scanner 162 and producing a 2D image and/or a 3D model of at least a portion of the patient. The display unit 164 may be configured to display the resulting 2D image and/or 3D model.

The imaging system 160 may also be in communication with the navigation controller 140 of the surgical navigation system 110. The imaging system 160 may be configured to communicate via a wired and/or a wireless connection with the navigation controller 140. For example, the imaging system 160 may be configured to provide pre-operative and/or intra-operative image data, such as the resulting 2D image and/or 3D model of the patient, to the navigation controller 140. The navigation controller 140 may then provide the resulting 2D image and/or 3D model to the display unit 120. If the imaging system 160 is a CT imaging device, the imaging system 160 may provide the navigation controller 140 with CT image data. The navigation controller 140 may store the 2D image, the 3D model, and/or the CT image data provided by the imaging system 160 in the memory M.

The imaging system 160 and/or the navigation controller 140 may be configured to perform image segmentation on patient images. For example, in an instance where the imaging system 160 images patient anatomy and generates CT imaging data, the navigation controller 140 may perform image segmentation on the CT imaging data to generate a volumetric image including a volumetric representation of the patient anatomy. In some instances, the volumetric representation of the patient anatomy may be a triangulated mesh of the patient anatomy. The navigation controller 140 may store the volumetric image in the memory M.

The surgical system 100 also includes a surgical instrument assembly 170 in wired or wireless communication with the navigation controller 140 directly, or indirectly. While only the first surgical instrument assembly 170 is illustrated in FIG. 1, it should be understood that it is only an exemplary configuration of the surgical system 100, and that it is contemplated that any number of surgical instrument assemblies may be positioned within the operating room. The surgical instrument assembly 170 includes a surgical instrument 172 including an end-effector 174 and a tracking device 176. The tracking device 176 includes a plurality of markers that are capable of being identified and/or tracked by the surgical navigation system 110. Reliable tracking of surgical instruments during the execution of surgical procedures to follow the planned surgical pathway and/or to avoid critical anatomical structures is of the utmost importance. Furthermore, providing feedback and/or notifying the user executing the procedure when the surgical instrument becomes misaligned with the surgical pathway and/or is at risk of impinging on a critical anatomical structure is of similar importance. The surgical instrument 172 may be coupled to a drill chuck, a tap for creating threads on the interior surface of a hole or aperture, a driver for driving or inserting a screw within the borehole or aperture of the bone, or another end effector. The surgical instrument assembly 170 may each be like any of those described in Intl. Patent Publication No. 2021/062373, which is hereby incorporated by reference in its entirety. The surgical system may, in addition or as an alternative to the surgical instrument assembly 170, include a surgical robot, such as the robotic manipulator described in U.S. Patent No. 11,033,341, which is hereby incorporated by reference.

Further, the navigation system 110 may include the tracking unit 112 to track the instrument assembly 170, the surgical robot, and/or other elements of the surgical system 100. The tracking unit 112 may include one or more sensors 114 for tracking the tracking device 176 of the surgical instrument assembly 170. The sensors may include cameras, such as CCD cameras, CMOS cameras, and/or optical image cameras, magnetic sensors, radio frequency sensors, or any other sensor adapted to detect and/or sense the position of a tracking device 176 of the surgical instrument assemblies 170. Description of a suitable tracking unit, and the various localizers that it can utilize may be found in U.S. Patent Publication No. 2017/0333137, which is hereby incorporated by reference in its entirety.

### II. Alert Zone Visualization

The navigation controller 140 may be configured to define alert zones corresponding to a patient anatomy. Alert zones may include areas of a patient anatomy that should not be contacted by a medical device during a medical procedure. During a medical procedure, a user may be warned to not contact anatomical structures within a defined alert zone. In one such instance, the alert zones may include critical anatomical features such as cortical walls, nerves, blood vessels or similar critical anatomical structures that should not be contacted during a medical procedure.

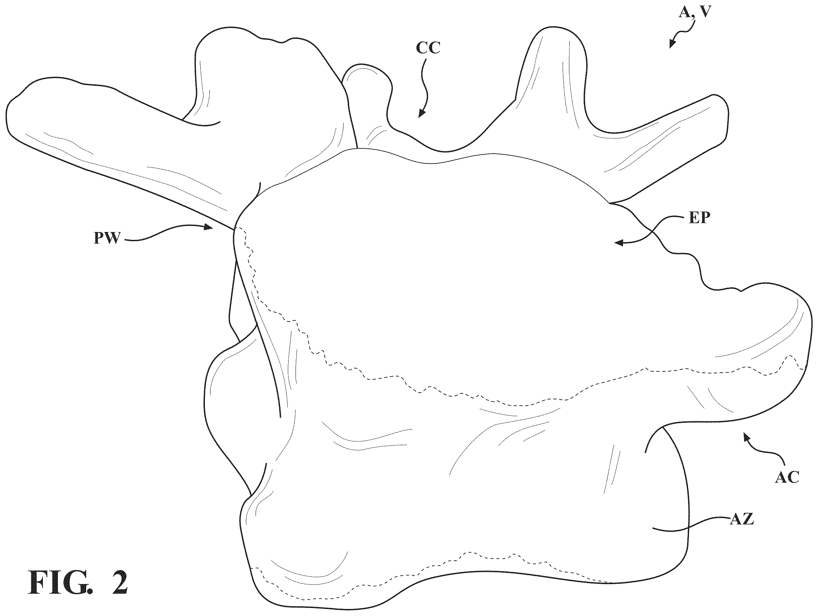

An example alert zone AZ is shown in FIG. 2. In the instance of FIG. 2, the patient anatomy A is a vertebra V and a portion of the anterior cortex AC of the vertebra V is defined as the alert zone AZ. During a spinal procedure where the vertebra V is treated and pedicle screws are inserted, a user may be warned to not contact the portion of the anterior cortex AC included by the alert zone AZ during the spinal procedure (e.g. during burring and/or insertion of the pedicle screws).

The alert zone AZ may be defined for any suitable anatomical structure. For example, the alert zone AZ may be defined for bone structure(s) of the knee, shoulder, elbow, ankle, or hip joint. Additionally, the alert zone AZ may be defined for any tissue treated during any type of medical procedure, including any spinal procedure, partial knee arthroplasty, total knee arthroplasty, total hip arthroplasty, anatomical shoulder arthroplasty, reverse shoulder arthroplasty, fracture repair surgery, osteotomies (e.g. peri-acetabular osteotomy, tibial osteotomy, and distal radius osteotomy), anterior cruciate ligament reconstruction, osteoid osteoma excision, bone tumor resection, fracture surgery, and the like.

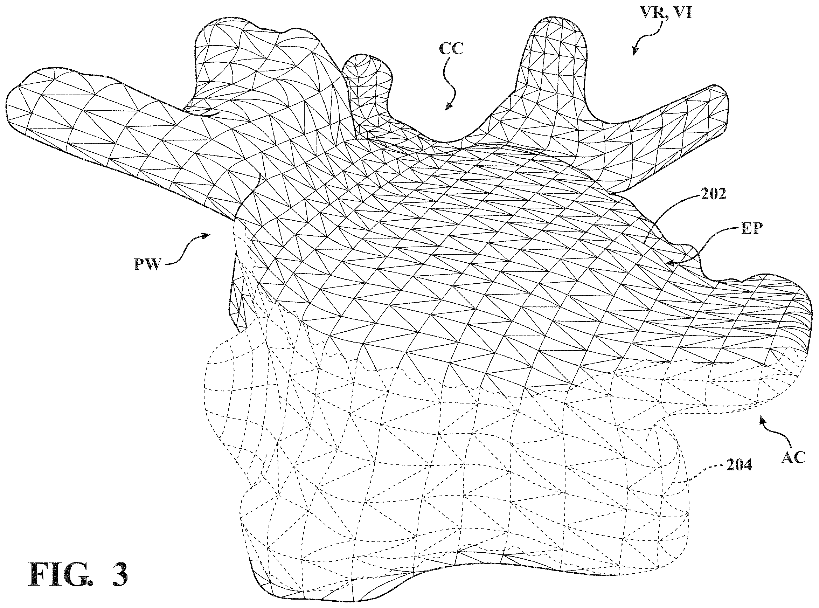

Referring to FIG. 3, a volumetric image VI is shown, with the volumetric image VI including a volumetric representation VR of the patient anatomy A. In the instance of FIG. 3, the volumetric image VI includes a volumetric representation VR of the vertebra V shown in FIG. 2. As previously stated, the imaging system 160 and/or the navigation controller 140 may be configured to perform image segmentation on patient images to generate a volumetric image VI of the patient anatomy A. For example, the navigation controller 140 may perform image segmentation on CT imaging data received from the imaging system 160 to generate the volumetric representation VR of the vertebra V. In the instance of FIG. 3, the volumetric representation VR of the vertebra V is illustrated as a triangulated mesh of the vertebra V.

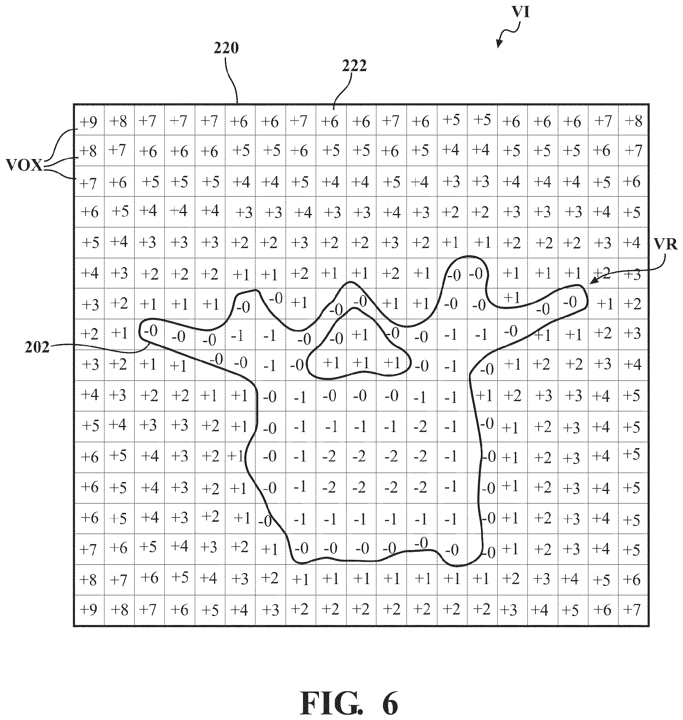

The volumetric image VI may be defined as including a plurality of voxels VOX. The plurality of voxels VOX are shown in FIG. 6 in two-dimensional form for illustrative purposes. Additionally, the volumetric image VI may include any suitable number of voxels for accurately representing the patient anatomy A.

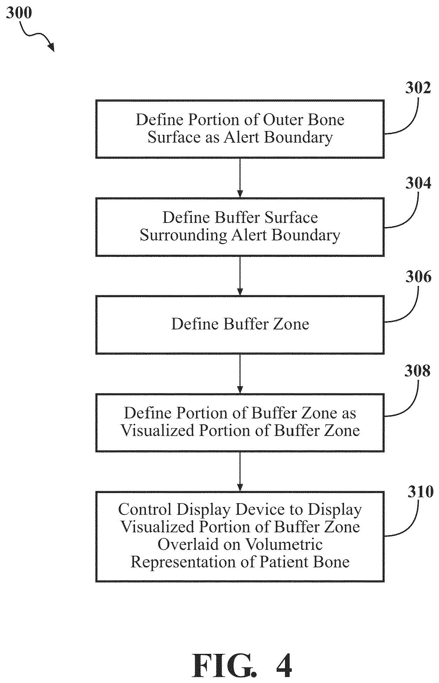

FIG. 4 illustrates a method 300 of defining and displaying the alert zone AZ on the display device 120 is shown. As shown, the method includes a step 302 of defining at least a portion of an outer surface of the volumetric representation VR of the patient anatomy A as an alert boundary; a step 304 of defining a buffer surface surrounding the alert boundary and offset from the alert boundary by a predefined buffer distance; a step 306 of defining a buffer zone as a portion of the volumetric image VI enclosed by the buffer surface; a step 308 of defining at least a portion of the buffer zone as a visualized portion of the buffer zone, wherein the visualized portion of the buffer zone intersects the volumetric representation VR of the patient anatomy A, and a step 310 of controlling the display device to display at least a portion of the visualized portion of the buffer zone overlaid on at least a portion of the volumetric representation VR of the patient anatomy A.

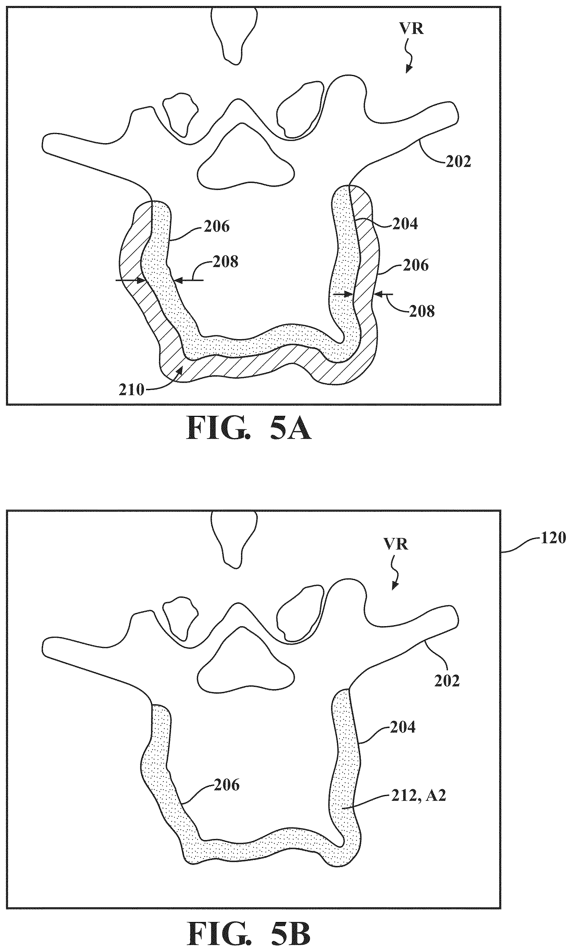

FIGS. 5A-5B illustrate an example operation of the method 300. First, the navigation controller 140 is configured to execute steps 302-308 of the method 300 to define the alert zone AZ. As shown in FIG. 5A, during definition of the alert zone AZ, the navigation controller 140 defines an alert boundary 204 and a buffer surface 206 offset from the alert boundary 204 by a predefined buffer distance 208, with the buffer surface 206 forming a buffer zone 210. The alert zone AZ is then defined as the portion 212 of the buffer zone 210 intersecting the volumetric representation VR of the patient anatomy A. The portion 212 of the buffer zone 210 is defined as the visualized portion 212, which is ultimately displayed by the display device 120 as the alert zone AZ. The navigation controller 140 then executes step 310 of the method 300 to display the alert zone AZ and the volumetric representation VR of the patient anatomy A on the display device 120, as shown in FIG. 5B.

The predefined buffer distance 208 may correspond to a size of the alert zone AZ. For example, in instances where a user prefers a larger alert zone AZ, the predefined buffer distance 208 may be defined using a larger value. Similarly, in instances where a user prefers a smaller alert zone AZ, the predefined buffer distance 208 may be defined using a smaller value. In some instances, the alert zone AZ may be determined to be of a default size and the predefined buffer distance 208 may be defined accordingly. In other instances, a size of the alert zone AZ may be set and/or manipulated by a user. In such instances, the predefined buffer distance 208 may be defined based on the size of the alert zone AZ as set/manipulated by the user.

The navigation controller 140 may be configured to perform the step 302 of defining at least a portion of an outer surface of the volumetric representation VR of the patient anatomy A as the alert boundary 204. As shown in FIG. 3, the volumetric representation VR of the vertebra V includes an outer surface 202, and a portion of the outer surface 202 is defined as the alert boundary 204. Referring to FIG. 2, the alert boundary 204 corresponds to the surface of the vertebra V within the alert zone AZ.

The alert boundary 204 may be identified by the navigation controller 140. For example, in some instances, the navigation controller 140 may be configured to identify a portion of the outer surface 202 corresponding to an anatomical landmark of the patient anatomy A and define the portion of the outer surface 202 corresponding to the anatomical landmark as the alert boundary 204. The navigation controller 140 may be configured to compare the volumetric representation VR of the patient anatomy A with exemplary templates of the patient anatomy A to identify anatomical landmarks. For example, the navigation controller 140 may be configured to compare the volumetric representation VR of the vertebra V with exemplary templates of the vertebra V to identify a central canal CC, pedicle walls PW, end plates EP, and an anterior cortex AC on the volumetric representation VR of the vertebra V. In the instance of FIG. 3, the navigation controller 140 identifies the anterior cortex AC and defines the portion of the outer surface 202 corresponding to the anterior cortex AC as the alert boundary 204.

In some instances, once the navigation controller 140 has identified anatomical landmarks on the volumetric representation VR of the vertebra V, a user may select an identified anatomical landmark to be included by the alert zone AZ. For example, the user may be configured to select an identified anatomical landmark via the GUI 150 displayed by the display device 120. The navigation controller 140 may define the portion of the outer surface 202 corresponding to the selected anatomical landmark as the alert boundary 204.

The alert boundary 204 may be identified by the user. For example, in some instances, the user may identify the alert boundary 204 via the user input devices 130 and/or the GUI 150. In one such instance, the display device 120 may display a GUI 150 including the volumetric representation VR of the patient anatomy A, and the user may then interact the GUI 150 via user input devices 130 to manually draw/outline/highlight portions of the outer surface 202 corresponding to the alert boundary 204. The navigation controller 140 then defines the portions of the outer surface 202 corresponding to drawn/outlined portions as the alert boundary 204.

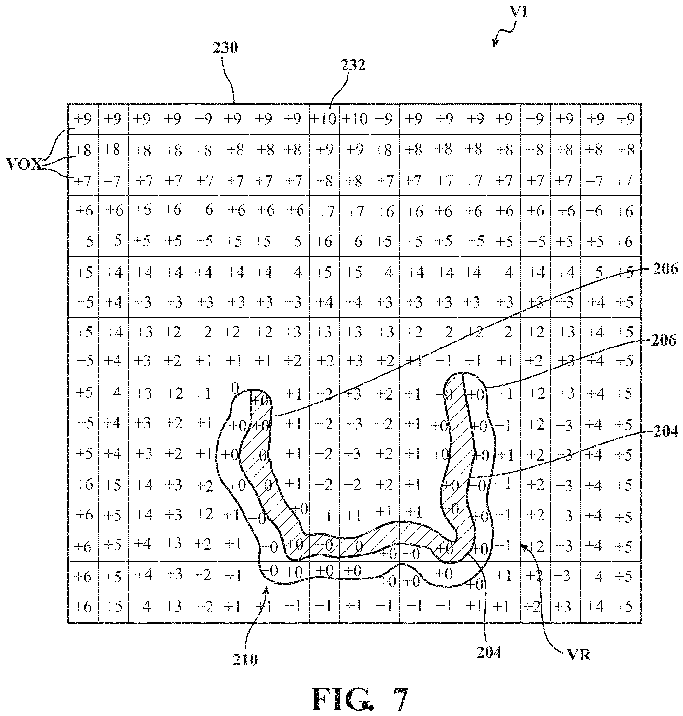

The navigation controller 140 may be configured to perform the steps 304-308 of the method by generating an anatomy distance field 222, shown in FIG. 6, and an alert zone distance field 232, shown in FIG. 7.

The anatomy distance field 220 generated by the navigation controller 140 may correspond to the volumetric image VI. As shown in FIG. 6, the anatomy distance field 220 includes a plurality of anatomy distance values 224, each anatomy distance value 224 being assigned to a voxel VOX of the volumetric image VI. The navigation controller 140 may be configured to assign signed anatomy distance values 224 to the voxels of the volumetric image VI. For example, the navigation controller 140 may be configured to identify voxels VOX of the volumetric image VI within the outer surface 202 of the volumetric representation VR and assign negative anatomy distance values 224 to the identified voxels VOX. The navigation controller 140 may also assign positive anatomy distance values 224 to the voxels VOX outside of the outer surface 202. Additionally, a magnitude of the anatomy distance values 224 assigned to each voxel VOX may be based on a distance of the voxel VOX from the outer surface 202. As the distance increases between the voxel VOX and the outer surface 202, a magnitude of the anatomy distance value 224 assigned to the voxel VOX also increases.

Similarly, the alert zone distance field 230 generated by the navigation controller 140 may correspond to the volumetric image VI. As shown in FIG. 7, the alert zone distance field 230 includes a plurality of alert zone distance values 234, each alert zone distance value 234 being assigned to a voxel VOX of the volumetric image VI. The navigation controller 140 may be configured to assign unsigned alert zone distance values 234 to the voxels of the volumetric image VI. For example, the navigation controller 140 may be configured to assign positive alert zone distance values 234 to each voxel VOX. Additionally, a magnitude of the alert zone distance values 234 assigned to each voxel VOX may be based on a distance of the voxel VOX from the alert boundary 204. As the distance increases between the voxel VOX and the alert boundary 204, a magnitude of the alert zone distance value 234 assigned to the voxel VOX also increases.

The anatomy distance values 224 and the alert zone distance values 234 may be any real number. The anatomy distance values 224 and the alert zone distance values 234 are illustrated in FIGS. 6 and 7 as whole numbers for illustrative purposes. In some instances, the anatomy distance values 224 and the alert zone distance values 234 may be numbers with a decimal point. Referring to FIG. 6, the anatomy distance values 224 that are within the outer surface 202 and close to the outer surface 202 are marked illustratively with "-0" to indicate a negative real number close to "0", such as "-0.01". Similarly, referring to FIG. 7, the alert zone distance values 234 that are close the alert boundary 204 are marked illustratively with "+0" to indicate a positive real number close to "0", such as "0.01". Advantageously, by using real numbers as for the anatomy distance values 224 and the alert zone distance values 234, the navigation controller 140 is able to perform interpolation on the anatomy distance values 224 and the alert zone distance values 234 to accurately determine a shape of the outer surface 202 and a shape of the surfaces of the alert zone AZ.

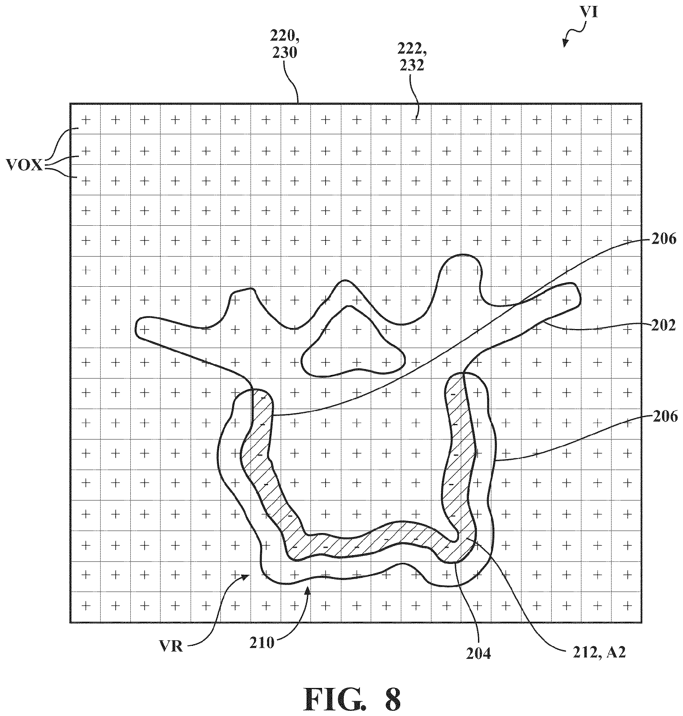

The navigation controller 140 may be configured to perform the steps 304-308 of the method by analyzing the anatomy distance value 224 and the alert zone distance field value 234 assigned to each voxel VOX. For example, the navigation controller 140 may be configured to perform the step 308 of defining the visualized portion 212 based on determining which voxels VOX of the volumetric image VI are assigned both a negative anatomy distance value 224 and an alert zone distance value 234 less than a threshold value. In other words, the navigation controller 140 may be configured to define the visualized portion 212 based on determining voxels VOX of the volumetric image VI including a negative anatomy distance value 224 and a positive alert zone distance value 234 less than a threshold value. Referring to FIG. 8, such voxels VOX are marked illustratively with a "-" and form the visualized portion 212.

The navigation controller 140 may be configured to perform step 304 of defining the buffer surface 206 and step 306 of defining the buffer zone 210 as part of step 308 of defining the visualized portion 212. For example, as previously stated, during step 308, the navigation controller 140 may define the visualized portion 212 based on determining which voxels VOX are assigned both a signed anatomy distance value 224 and an unsigned alert zone distance value 234 less than a threshold value. The navigation controller 140 may define the buffer surface 206 and the buffer zone 210 by determining which voxels VOX are assigned an alert zone distance value 234 less than a threshold value. The threshold value may be based on the predefined buffer distance 208 shown in FIG. 5A. As such, by determining which voxels VOX are assigned an alert zone distance value 234 less than a threshold value, the navigation controller 140 is able to define the buffer surface 206, as well as the buffer zone 210.

The navigation controller 140 may also be configured to determine which voxels are not a part of the visualized portion 212 of the buffer zone 210. For example, referring to FIG. 8, voxels VOX marked illustratively with a "+" are determined to be outside the visualized portion 212. Referring to FIGS. 6-7, voxels VOX that are assigned both a negative anatomy distance value 224 and an alert zone distance value 234 greater than the threshold value correspond to voxels VOX that are within the outer surface 202, but outside the buffer zone 210. Such voxels do not form the visualized portion 212 and marked illustratively with a "+" in FIG. 8. Additionally, referring to FIGS. 6-7, voxels VOX that are assigned both a positive anatomy distance value 224 and an alert zone distance value 234 less than the threshold value correspond to voxels VOX that are within the buffer zone 210, but outside the outer surface 202. Such voxels also do not form the visualized portion 212 and marked illustratively with a "+" in FIG. 8. Furthermore, referring to FIGS. 6-7, voxels VOX that are assigned both a positive anatomy distance value 224 and an alert zone distance value 234 greater than the threshold value correspond to voxels VOX that are not within the outer surface 202 and not within the buffer zone 210. Such voxels also do not form the visualized portion 212 and marked illustratively with a "+" in FIG. 8.

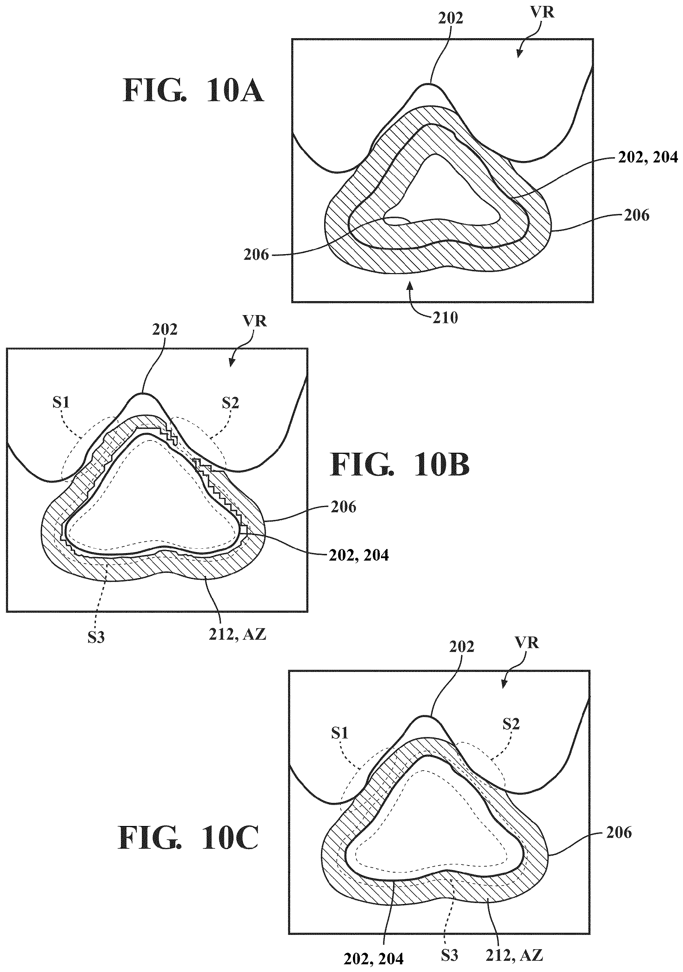

In more specific instances, the navigation controller 140 may be configured to calculate an interpolation value for each voxel VOX. The navigation controller 140 may then perform interpolation on these interpolation values to define the visualized portion 212 with a continuous outline such that the alert zone AZ is ultimately displayed with a continuous outline. Referring to FIG. 10A, a buffer zone 210 is shown, where the buffer zone 210 is defined as including a buffer surface 206 offset from an alert boundary 204, where a portion of the outer surface 202 of the central canal CC is identified as the alert boundary 204. The navigation controller 140 defines the visualized portion 212 as the portion of the buffer zone 210 that is within the outer surface 202 and within the buffer zone 210. FIG. 10B provides one instance of defining the visualized portion 212, where the navigation controller 140 omits voxels VOX outside the outer surface 202 during definition of the visualized portion 212. However, some voxels VOX may be identified as being outside the outer surface 202, while including some portions within the outer surface 202. As a result, by omitting voxels VOX outside the outer surface 202, the navigation controller 140 defines the visualized portion 212 as including a pixelized outline, which is not continuous. Such pixelization is shown in sections S1, S2, S3 of FIG. 10B. Referring now to FIG. 10C, the navigation controller 140 calculates an interpolation value for each voxel VOX, where the interpolation value is based on the anatomy distance value 224 and the alert zone distance field value 234 assigned to each voxel VOX. Specifically, for voxels VOX within the outer surface 202 and the buffer zone 210 (i.e. voxels VOX that are assigned both a signed anatomy distance value 224 and an unsigned alert zone distance value 234 less than a threshold value), the navigation controller 140 calculates the interpolation value based on the anatomy distance value 224 for voxels VOX near/outside the outer surface 202 and based on the alert zone distance value 234 for voxels VOX further from the outer surface 202. By calculating interpolation values based on the assigned anatomy distance values 224 for voxels VOX near/outside the outer surface 202, the navigation controller 140 is able to define the outline of the visualized portion 212 with a continuous outline, as shown in sections S1, S2, S3 of FIG. 10C. More particularly, as previously stated, the navigation controller 140 may perform interpolation on the anatomy distance values 224 to accurately determine a shape of the outer surface 202. As such, because the navigation controller 140 calculates the interpolation value based on the anatomy distance value 224 for voxels VOX within the buffer zone 210 and near/outside the outer surface 202, when the navigation controller 140 performs interpolation on these interpolation values, the navigation controller 140 defines the outline of the visualized portion 212 based on the anatomy distance values 224 to define a continuous outline for the visualized portion 212 that corresponds to the shape of the outer surface 202.

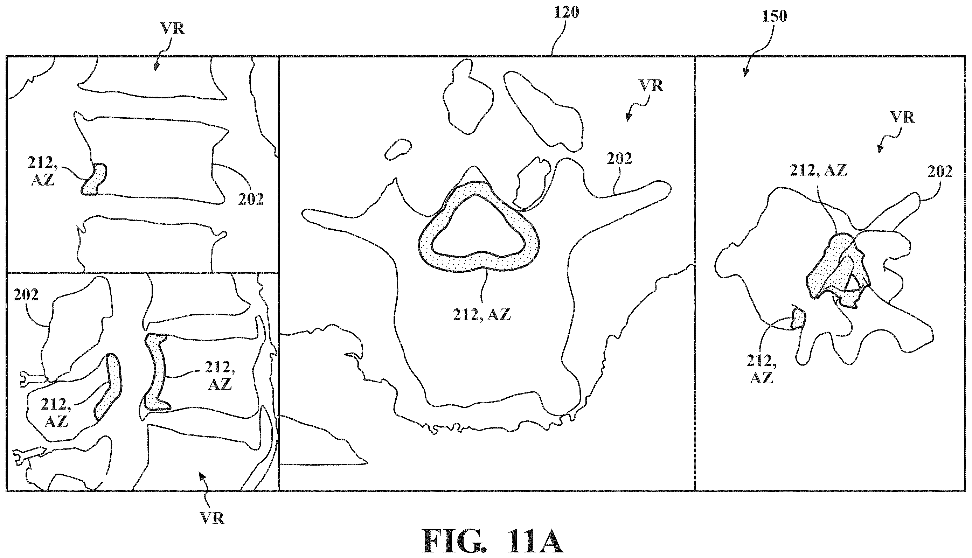

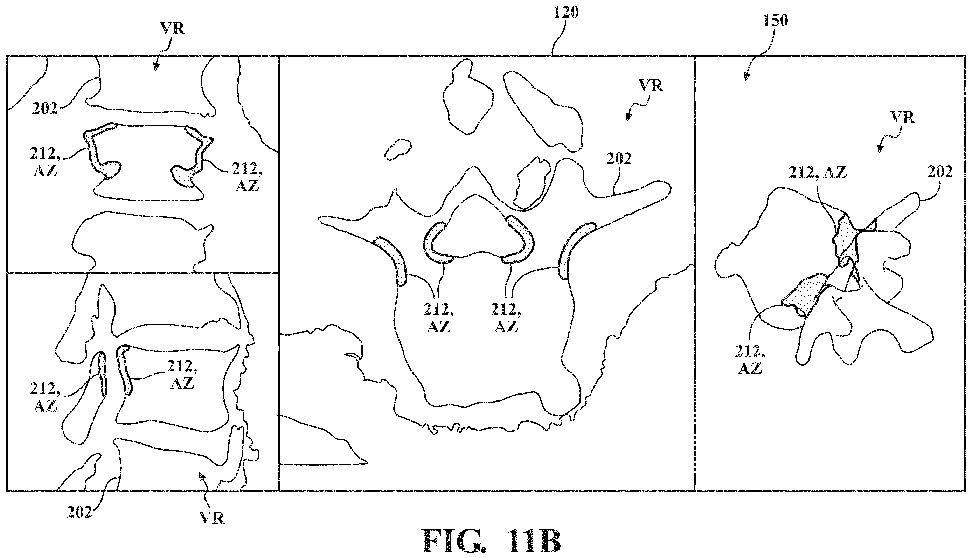

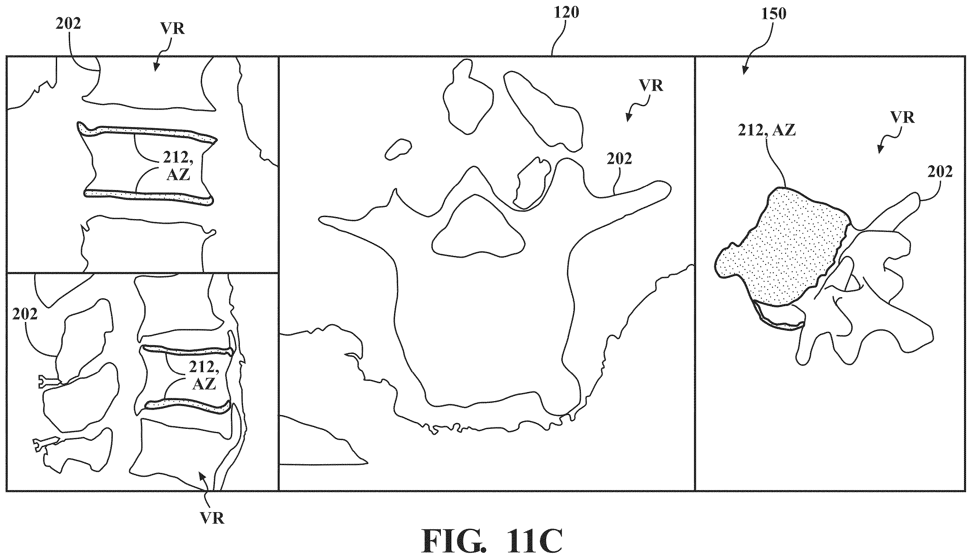

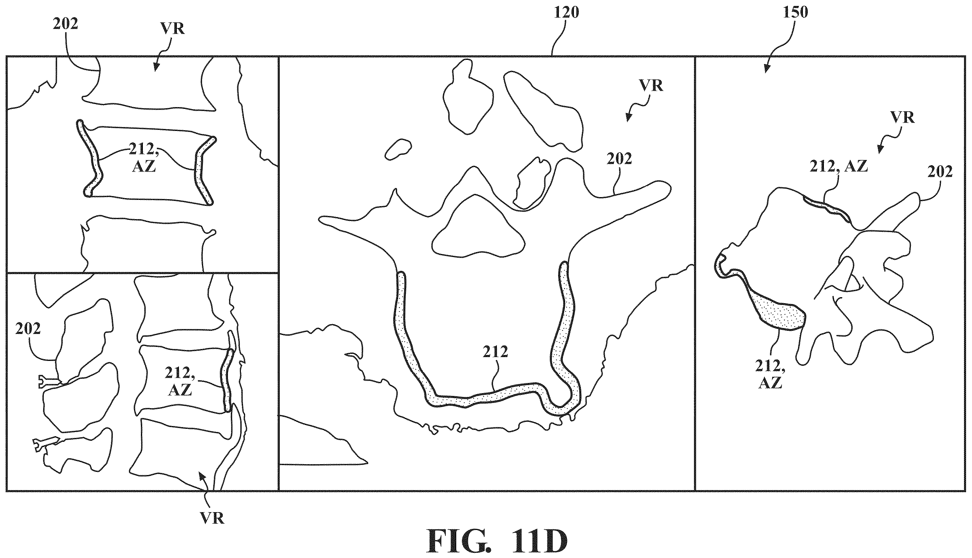

Once the navigation controller 140 has defined the visualized portion 212 of the buffer zone 210, the navigation controller 140 may be configured to display the visualized portion 212 on the display device 120 as the alert zone AZ. For example, the navigation controller 140 may display at least a portion of the visualized portion 212 overlaid on at least a portion of the volumetric representation VR of the patient anatomy A. In the instance of FIG. 11A, the visualized portion 212 corresponds to the central canal CC of the vertebra V, and the visualized portion 212 is displayed as the alert zone AZ and overlaid on the volumetric representation VR of the vertebra V. In the instance of FIG. 11B, the visualized portion 212 corresponds to the pedicle walls PW of the vertebra V, and the visualized portion 212 is displayed as the alert zone AZ and overlaid on the volumetric representation VR of the vertebra V. In the instance of FIG. 11C, the visualized portion 212 corresponds to the end plate EP of the vertebra V, and the visualized portion 212 is displayed as the alert zone AZ and overlaid on the volumetric representation VR of the vertebra V. In the instance of FIG. 11D, the visualized portion 212 corresponds to the anterior cortex AC of the vertebra V, and the visualized portion 212 is displayed as the alert zone AZ and overlaid on the volumetric representation VR of the vertebra V. Additionally, as shown in FIGS. 11A-11D, the display device 120 may be various perspective and cutaway views of the volumetric representation VR of the vertebra V and the alert zone AZ via the GUI 150.

It will be appreciated that although the method is described above with reference to CT image data and the spine of the patient, the method may also be applied to other forms of 3D image data and other tissues of the patient. In one example, the method may utilize MRI image data or the like. In another example, the method may be used to segment a joint of the patient, such as a knee joint, or a hip joint. Other alterations to the method are contemplated. The described system and method may be useful for a variety of orthopaedic joint procedures (for example replacement of hip, knee, shoulder, ankle and elbow joints), peri-acetabular osteotomy, tibial osteotomy, distal radius osteotomy, anterior cruciate ligament reconstruction, osteoid osteoma excision, bone tumor resection, spinal procedures (for example in the placement of pedicle screws), and fracture surgery.

The methods in accordance with the present teachings is for example a computer implemented method. For example, all the steps or merely some of the steps (i.e. less than the total number of steps) of the method in accordance with the present teachings can be executed by a computer (for example, at least one computer). A configuration of the computer implemented method is a use of the computer for performing a data processing method. Further, in the present teachings, the methods disclosed herein comprise executing, on at least one processor of at least one computer (for example at least one computer being part of the navigation system), the following exemplary steps which are executed by the at least one processor.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A surgical navigation system (110) comprising:
a display device (120);
a memory device (M) containing a volumetric image (VI), the volumetric image (VI) including a volumetric representation (VR) of a patient anatomy, the volumetric representation (VR) of the patient anatomy having an outer surface; and
a controller (140) in communication with the display and the memory device (M), the controller (140) configured to:
define at least a portion of the outer surface as an alert boundary (204);
define a buffer surface (206) surrounding the alert boundary (204) and offset from the alert boundary (204) by a predefined buffer distance (208);
define a buffer zone (210) as a portion of the volumetric image (VI) enclosed by the buffer surface (206);
define at least a portion of the buffer zone (210) as a visualized portion (212) of the buffer zone (210), wherein the visualized portion (212) of the buffer zone (210) intersects the volumetric representation (VR) of the patient anatomy; and
control the display device (120) to display at least a portion of the visualized portion (212) of the buffer zone (210) overlaid on at least a portion of the volumetric representation (VR) of the patient anatomy.

2. The surgical navigation system (110) of claim 1, wherein the controller (140) is configured to generate the volumetric representation (VR) of the patient anatomy by performing segmentation on a CT image of the patient anatomy.

3. The surgical navigation system (110) of claim 1 or 2, further comprising a user input device (130) configured to receive an identification of the alert boundary (204).

4. The surgical navigation system (110) of claim 3, wherein the user input device (130) is configured to receive a selected portion of the outer surface as the identification of the alert boundary (204), and wherein the controller (140) is configured to define the selected portion of the outer surface as the alert boundary (204).

5. The surgical navigation system (110) of any one of claims 1 to 4, wherein the controller (140) is further configured to identify a portion of the outer surface corresponding to an anatomical landmark of the patient anatomy.

6. The surgical navigation system (110) of claim 5, wherein the controller (140) is configured to define the portion of the outer surface corresponding to the anatomical landmark of the patient anatomy as the alert boundary (204).

7. The surgical navigation system of any one of claims 1 to 6, wherein the volumetric image (VI) includes a plurality of voxels (VOX).

8. The surgical navigation system (110) of claim 7, wherein the controller (140) is configured to generate an anatomy distance field (220) corresponding to the volumetric image (VI), wherein the anatomy distance field (220) includes a plurality of anatomy distance values (224), each anatomy distance value (224) being assigned to a voxel (VOX) of the volumetric image (VI).

9. The surgical navigation system (110) of claim 7 or 8, wherein the controller (140) is configured to identify the voxels (VOX) of the volumetric image (VI) within the outer surface.

10. The surgical navigation system (110) of claim 8 or 9, wherein the controller (140) is configured to assign a signed anatomy distance value (224) to a voxel (VOX) of the volumetric image (VI) within the outer surface.

11. The surgical navigation system (110) of any one of claims 8 to 10, wherein the controller (140) is configured to generate an alert zone distance field (230) corresponding to the volumetric image (VI), wherein the alert zone distance field (230) includes a plurality of alert zone distance values (234), each alert zone distance value (234) being assigned to a voxel (VOX) of the volumetric image (VI).

12. The surgical navigation system (110) of claim 11, wherein the controller (140) is configured to assign an unsigned alert zone distance value (234) to a voxel (VOX) of the volumetric image (VI) based on a distance of the voxel (VOX) from the alert boundary (204).

13. The surgical navigation system (110) of claim 12, wherein the controller (140) is configured to define the visualized portion (212) of the buffer zone (210) based on determining voxels (VOX) of the volumetric image (VI) assigned a signed anatomy distance value (224) and an unsigned alert zone distance value (234) less than a threshold value.

14. The surgical navigation system (110) of claim 13, wherein the threshold value is based on the predefined buffer distance (208).

15. A method of operating a surgical navigation system (110) including a display device (120), the method comprising steps of:
generating a volumetric image (VI) including a volumetric representation (VR) of a patient anatomy, the volumetric representation (VR) of the patient anatomy having an outer surface;
defining at least a portion of the outer surface as an alert boundary (204),
defining a buffer surface (206) surrounding the alert boundary (204) and offset from the alert boundary (204) by a predefined buffer distance (208),
defining a buffer zone (210) as a portion of the volumetric representation (VR) of the patient anatomy enclosed by the buffer surface (206),
defining at least a portion of the buffer zone (210) as a visualized portion (212) of the buffer zone (210), wherein the visualized portion (212) of the buffer zone (210) intersects the volumetric representation (VR) of the patient anatomy; and
displaying, with the display device (120), at least a portion of the visualized portion (212) of the buffer zone (210) overlaid on at least a portion of the volumetric representation (VR) of the patient anatomy.
